# EUROPEAN PATENT SPECIFICATION

(11) **EP 1 100 476 B1**
(45) Date of publication and mention of the grant of the patent: **16.06.2004**
(21) Application number: 99935613.2
(22) Date of filing: 16.07.1999
(51) Int. Cl.: A61K 9/70, A61K 31/55, A61P 9/12

(54) **FORMULATIONS FOR THE TRANSDERMAL ADMINISTRATION OF FENOLDOPAM**
FORMULIERUNGEN ZUR TRANSDERMALEN VERABREICHUNG VON FENOLDOPAM
FORMULATIONS POUR L'ADMINISTRATION TRANSDERMIQUE DE FENOLDOPAM

(30) Priority: 24.07.1998 US 94059 P
(43) Date of publication of application: 23.05.2001
(73) Proprietor: ALZA Corporation, Mountain View, CA 94039-7210 (US)
(72) Inventor: VAN OSDOL, William, W., Mountian View, CA 94040 (US); CRISOLOGO, Nieves, M., Sunnyvale, CA 94086-3042 (US); YUM, Su, Il, Los Altos, CA 94024 (US)
(74) Representative: Suèr, Steven Johannes
(86) International application number: PCT/US1999/016083
(87) International publication number: WO 2000/004886

(56) References cited:
- WO-A-91/16041
- WO-A-99/15210
- US-A- 4 197 297
- US-A- 5 656 286
- US-A- 5 719 197
- US-B- 5 656 286
- US-B- 5 719 197

## Description

### FIELD OF INVENTION

This invention relates to sustained release formulations for the safe and efficacious administration of fenoldopam for, among other things, the treatment of hypertension, congestive heart failure, and acute and chronic renal failure. More particularly, the invention relates to novel methods, compositions, and devices for transdermally administering fenoldopam transdermally to a subject over a sustained time period.

### BACKGROUND OF THE INVENTION

The transdermal route of parenteral delivery of drugs and other biologically active agents ("agents") has been proposed for a wide variety of systemically acting and locally acting agents on either a rate-controlled or non-rate-controlled basis and is described in numerous technical publications such as the following: U.S. Patent Nos. 3,598,122; 3,598,123; 3,731,683; 3,797,494; 4,031,894; 4,201,211; 4,286,592; 4,314,557; 4,379,454; 4,435,180; 4,559,222; 4,573,995; 4,588,580; 4,645,502; 4,698,062; 4,704,282; 4,725,272; 4,781,924; 4,788,062; 4,816,258; 4,849,226; 4,904,475; 4,908,027; 4,917,895; 4,938,759; 4,943,435; 5,004,610; 5,071,656; 5,122,382; 5,141,750; 5,284,660; 5,314,694; 5,342,623; and 5,635,203, the disclosures of which are incorporated in their entirety herein by reference.

When first investigated in depth in the late 1960's, the transdermal route of administration appeared to offer many advantages, particularly with respect to agents that had short half lives and therefore required frequent, repeated dosing or were subject to a high degree of first-pass metabolism by the liver. The peaks and valleys in blood concentration resulting from frequent periodic doses of short half-life agents would be eliminated and replaced by substantially constant plasma concentration. This would not only improve individual compliance but also would eliminate the alternating periods of high side-effects and ineffective blood concentrations associated with periodic dosing. Administering the agent through the skin directly into the blood stream would also eliminate first-pass metabolism of orally administered agents.

It was initially assumed, theoretically, that any short half-life agent of high potency and skin permeability would be suitable for safe and effective transdermal administration. This assumption, however, has not been proven true.

The failure of the transdermal route to fulfill the initial expectations of its potential as an administrative portal was primarily due to the incredible variety of properties with which nature has endowed the skin to permit it to perform its function as the primary barrier to prevent the ingress of foreign substances into the body. See Transdermal Drug Delivery: Problems and Possibilities, B. M. Knepp, et al, CRC Critical Reviews and Therapeutic Drug Carrier Systems, Vol. 4, Issue 1 (1987) and Transdermal Delivery Systems: A Medical Rationale, Gary W. Cleary, Topical Drug Bioavailabililty, Bioequivalence, and Penetration, Plenum Press, 1993. Thus, the transdermal route of administration, rather than being available to every short half-life agent of high potency and skin permeability, was found to be available only to those few agents that possess the proper combination of a host of characteristics, most of which are unpredictable, required to render the agent suitable for safe and effective transdermal administration.

The most significant of these characteristics are the following:
1. Skin Permeability. The permeability of the skin to the agent must be sufficiently high so that the agent can be administered at a therapeutically effective rate through an area of skin no greater than about 200 cm² and preferably no greater than 50 cm². The person-to-person variation in skin permeability at similar sites should also be considered. U.S. Patent Nos. 4,568,343, 4,746,515, 4,764,379,4,863,738, 4,865,848, 4,888,354, 4,900,555, 5,378,730, 5,629,019, 5,641,504, 5,686,097, and WO 95/09006, WO 95/01167, WO 96/37231, and WO 96/40259 are related to various compositions and methods for enhancing permeation of drugs through the skin and are hereby incorporated in their entirety by reference.
2. Skin Binding. The skin beneath the transdermal delivery device has the capability of creating a skin depot of drug by absorbing, adsorbing, or binding a certain amount of agent. The amount of agent so bound must be supplied to the skin before the agent can be delivered into the blood stream at steady, therapeutically effective rates. If large amounts of the agent are bound in the skin, significant delays in the onset of therapeutic effect ("lag time") will be observed together with corresponding delays in termination of effect upon removal of the device. The potential also exists for toxic quantities of potent agents to be contained within the skin beneath the device. Skin binding is not related to skin permeability. Agents that are highly permeable may also be highly bound causing a lag time sufficiently long as to render them unsuitable for their intended use.
3. Irritation. The skin reacts to many topically applied substances, particularly those maintained under occlusion, by blistering or reddening accompanied by unpleasant burning, itching, and stinging sensations. Animal models are used to screen for irritation. Animal models, however, often produce both false positives and false negatives. There is also a wide interpersonal variation in susceptibility to irritation. An agent must be minimally irritating in a large percentage of the target population in order to be suitable for safe and effective transdermal administration. U.S. Patent Nos. 4,552,872, 4,756,710, 5,028,431, 5,130,139, 5,160,741, 5,304,379, and 5,451,407 are directed to overcoming problems of skin irritation associated with transdermal drug delivery and are hereby incorporated in their entirety by reference.
4. Sensitization. Sensitization is an allergic reaction which is induced when an agent is first applied to the skin and is elicited upon continued exposure which may occur immediately or after a long period of seemingly harmless exposure.
   The sensitization may be local, elicited by topical exposure, which manifests itself as contact dermatitis accompanied by blistering, itching, reddening and burning at the site of application. More seriously, the sensitization may be systemic, elicited by topical application but manifesting itself by more general allergic reactions at sites other than the site of application. Most seriously, the systemic sensitization may be elicited by oral or intravenous administration of the drug. If the latter occurs, the individual will be unable to take the drug by any route of administration.
   Animal models are used to screen for sensitization. Animal models, however, produce both false positives and false negatives. There is also a wide variation in the allergic reaction among individuals as well as between sexes, races and skin types. It is obvious that a useful transdermal agent must be minimally sensitizing in a large percentage of the target population. U.S. Patent Nos. 5,000,956, 5,049,387, 5,120,145, and 5,149,539 are directed to overcoming sensitization problems associated with transdermal drug delivery by the coadministration of a corticosteroid and are hereby incorporated in their entirety by reference
5. Pharmacokinetic Properties. The half-life of an agent is the time after administration that half of the amount administered has been eliminated from the body. Because blood concentrations of continuously administered agents continue to increase for approximately five half-lives before steady-state constant blood concentrations are achieved, an agent must have a relatively short half-life to be suitable for continuous transdermal administration. The transdermal half-lives of most agents have not been determined. When half-lives of agents determined from intravenous administration are compared with half-lives determined from transdermal administration, the transdermal half-lives are generally longer but there can be wide variation in half-life between individuals based upon factors such as age, sex, health, and body type.
6. Pharmacodynamic Properties. Constant blood levels may not produce the desired therapeutic effects. For example, a therapeutic effect may only be observed at peak blood concentration obtained from bolus dosing but the peak blood or plasma concentration cannot be maintained because of side effects associated therewith. Also, continuous administration of many agents produces tolerance that may require either some agent-free interval or continually increasing and therefore potentially hazardous doses of the agent.
7. Potency. Although a certain degree of potency is required for transdermally administered agent to be effective, it is also possible for an agent to be too potent. As potency increases, lower blood concentrations are required and much smaller quantities are administered. Because of normal inter-individual variations and skin permeability, it may not be possible to precisely control whether a individual is receiving 1 µg/hr or 2 µg/hr, for example. For a highly potent agent, a 1 µg/hr administration may be totally ineffective and a 2 µg/hr rate fatal. Thus, the therapeutic index of an agent, which is the ratio of toxic blood concentration to the therapeutic blood concentration, becomes extremely significant. A highly potent agent should also have a relatively wide therapeutic window in order to be suitable for transdermal administration.
8. Metabolism. One of the perceived advantages of transdermal administration was that it avoided the "first-pass" metabolism of the agent by the liver that is associated with oral administration. It has now been recognized, however, that the skin is also a large metabolizing organ in the body for some drugs. Thus, although first-pass metabolism that occurs after an orally administered agent enters the blood stream can be avoided, skin metabolism, which occurs before the agent enters the bloodstream, cannot be avoided. Skin metabolism is capable of creating metabolites that are inactive, irritating, toxic, or comparable in biological activity to that of the agent. To be suitable for transdermal administration, an agent must have metabolic properties that are consistent with its therapeutic use on continuous administration.

The above summarizes the primary characteristics that effect suitability of an agent for transdermal administration that have been recognized to date. There are undoubtedly others, some of which have not yet been recognized, and, in order for an agent to be suitable for transdermal administration, it must possess the right combination of all these characteristics, a combination of which, as illustrated by the very few drugs that are now suitable for administration from transdermal delivery devices, is quite rare and unpredictable.

WO 91/16041 discloses sublingual dosage forms comprising starch waters. Fenoldopam is mentioned as a possible drug candidate for such dosage forms.

US 5656286 discloses a composition containing polyvinylpyrrolidone, adhesive, cosolvent, penetration enhancer and an active agent. Fenoldopam is mentioned as one possible active agent.

US 5719197 discloses a composition comprising an active agent, a bioadhesive carrier and a solvent. Fenoldopam is mentioned as one possible active agent.

The present invention is directed to the transdermal administration of fenoldopam, 6-Chloro-2,3,4,5-tetrahydro-1-(4-hydroxyphenol)-1H-3-benzazapine-7,8 diol for the treatment of, among others, hypertension, congestive heart failure, and acute and chronic renal failure. Fenoldopam (Corlopam®) is a renal vasodilator DA1 agonist that produces dose-dependent reduction in systolic and diastolic blood pressure without producing clinically significant increases in heart rate. The elimination half-life of fenoldopam is about 5 minutes in mild to moderate hypertensive patients, with little difference between the R (active) and S isomers. The preparation of fenoldopam is described in U.S. Patent Nos. 4,197,297, 4,321,195 and 4,705,862, which are hereby incorporated in their entirety by reference.

Currently, fenoldopam is administered by infusion at a maximum rate of up to 1.6µg/kg min for periods of up to 48 hours. Oral administration does not provide any clinical benefit, thus transdermal administration offers several advantages. For example, transdermal administration of fenoldopam significantly enhances patient compliance by alleviating the discomfort of needles and cumbersome I.V. apparatii by providing a convenient dosage form for once or twice weekly application. Other benefits discussed above associated with the transdermal administration of fenoldopam are also provided, such as sustained blood levels.

### DESCRIPTION OF TERMS

As used herein, the term "fenoldopam" intends not only the basic form of fenoldopam but also pharmaceutically acceptable salt forms of fenoldopam, the R or S enantiomers of fenoldopam, either individually or as a racemic mixture, and to mixtures thereof.

As used herein, the term "fenoldopam therapy" intends all medical conditions for which fenoldopam is or will be indicated, including, without limitation, for the treatment of hypertension, congestive heart failure, and acute and chronic renal failure.

As used herein, the term "individual" intends a living mammal and includes, without limitation, humans and other primates, livestock and sports animals such as cattle, pigs and horses, and pets such as cats and dogs.

As used herein, the term "monoglyceride" refers to a monoglyceride or mixture of monoglycerides of C₈₋₂₀ fatty acids and includes, without limitation, glycerol monolaurate (GML), glycerol monooleate (GMO), glycerol monocaprate (GMC), glycerol monocaprylate (GMCL), and glycerol monolinoleate (GMLO).

As used herein, the term "permeation enhancement" intends an increase in the permeability of skin to fenoldopam in the presence of a permeation enhancer as compared to permeability of skin to fenoldopam in the absence of a permeation enhancer.

As used herein, the term "permeation enhancer" intends an agent or a mixture of agents which acts to increase the permeability of the skin to fenoldopam.

As used herein, the term "permeation-enhancing amount" intends an amount of a permeation enhancer which provides permeation enhancement throughout a substantial portion of the administration period.

As used herein, the phrase "predetermined area of skin" intends a defined area of intact unbroken skin tissue. That area will usually be in the range of about 5 cm² to about 100 cm².

As used herein the term "salt" intends, but is not limited to, pharmaceutically acceptable organic or inorganic salts. Typical inorganic salts include hydrogen halides such as hydrochlorides, carbonates, phosphates, sulfates, hydrogen sulfates, hydrobromides, nitrates, and sulfides. Organic salts include, but are not limited to, acid addition salts including salts of monocarboxylic and polycarboxylic acids such as acetic acid, malic acid, maleic acid, propionic acid, succinic acid, fumaric acid, citric acid, benzoic acid, cinnamic acid, tartaric acid, and the like.

As used herein, the phrase "sustained time period" or "administration period" intends at least about 8 hours and will typically intend a period in the range of about one to about seven days.

As used herein, the term "therapeutically effective amount" intends the dose of fenoldopam and/or its active metabolites that provides fenoldopam therapy, in the case of adult and juvenile humans, the dosage range is about 1-20 mg fenoldopam per day.

As used herein, the term "therapeutically effective rate" intends a delivery rate of fenoldopam and/or its active metabolites effective to achieve therapeutic blood or plasma levels in an individual during the administration period and is typically within the range of about 0.01-1.6 µg/kg/min.

As used herein, the term "therapeutic blood or plasma level" intends the level of fenoldopam and/or its active metabolites in blood or plasma that achieves a therapeutic effect for the desired fenoldopam therapy. For individuals with mild to moderate malignant hypertension, this range is about 1-10 ng/mL.

As used herein, the term "transdermal" intends percutaneous administration, i.e. passage of fenoldopam through intact unbroken skin into the systemic circulation.

### SUMMARY OF THE INVENTION

It is an object of this invention to provide sustained release formulations to administer a therapeutically effective amount of fenoldopam and/or its active metabolites, over an administration period.

More specifically, it is an object of this invention to provide compositions for the transdermal delivery of fenoldopam and/or its active metabolites, and delivery systems for effecting the same, which are suitable for the transdermal administration of fenoldopam and/or its active metabolites continuously through skin at a therapeutically effective rate in order to achieve and maintain therapeutic blood or plasma levels in an individual.

Another object of this invention is to improve the compliance of patients in need of fenoldopam therapy by providing compositions and devices for the transdermal administration of fenoldopam at a therapeutically effective rate.

It has been discovered that fenoldopam can be safely and efficaciously administered transdermally at a therapeutically effective rate to provide, among other things, treatment for hypertension, congestive heart failure, and acute renal failure when coadministered with a suitable permeation enhancer.

According to one aspect of the present invention, there is provided a composition of matter for administration of fenoldopam through intact skin, the composition comprising fenoldopam and a skin permeation enhancer in a carrier, the skin permeation enhancer being present in an amount sufficient to enhance transdermal flux of fenoldopam compared to transdermal flux of fenoldopam from a similar composition having no skin permeation enhancer, the composition being effective for sustained delivery of fenoldopam through the skin at a therapeutically effective rate, characterized in that the composition comprises:
(a) 1 to 50 weight % of fenoldopam;
(b) 1 to 50 weight % of the skin permeation enhancer; and
(c) 30 to 90 weight % of a polymeric carrier.

The permeation enhancer may be any permeation enhancer known in the art to increase permeability of drugs through skin and includes, but is not limited to, those disclosed in the above cited patents. The permeation enhancer may comprise, but is not limited to, monoglycerides, C₁₀-C₂₀ fatty acid esters including ethyl palmitate and isopropyl myristate; acyl lactylates such as caproyl lactylic acid and lauroyl lactylic acid; dimethyl lauramide; dodecyl (lauryl) acetate; lactate esters such as lauryl lactate, and myristyl lactate; monoalkyl ethers of polyethyleneglycol and their alkyl or aryl carboxylic acid esters and carboxymethyl ethers such as polyethylene glycol-4 lauryl ether (Laureth-4) and polyethylene glycol-2 lauryl ether (Laureth-2); Myreth-3, myristyl sarcosine, and methyl laurate.

Preferably, the transdermal flux of fenoldopam is at least 1 *µ*g per hour per cm² of skin and the administration period is at least 6 hours.

Conveniently, the fenoldopam comprises a pharmaceutically acceptable salt. More preferably, the salt is fenoldopam mesylate.

In preferred embodiments, the permeation enhancer comprises a monoglyceride, myristyl sarcosine, or a mixture thereof.

Conveniently, the composition further comprises a cosolvent selected from fatty acid esters, caproyl lactylic acid, lauroyl lactylic acid, and dimethyl lauramide. Preferably, the monoglyceride is glycerol monolaurate and the cosolvent is selected from dodecyl acetate, lauryl lactate, isopropyl myristate, ethyl palmitate, and methyl laurate.

More preferably the composition comprises 5 to 40 weight % fenoldopam base and 5 to 50 weight % of a skin permeation enhancer comprising a monoglyceride and a fatty acid ester.

According to a further aspect of the present invention, there is provided a device for the transdermal administration of fenoldopam at a therapeutically effective rate, comprising:
(a) a reservoir comprising a composition comprising fenoldopam and a skin permeation enhancer in a carrier, the skin permeation enhancer being present in an amount sufficient to enhance transdermal flux of fenoldopam compared to transdermal flux of fenoldopam from a similar composition having no skin permeation enhancer, the composition being effective for sustained delivery of fenoldopam through the skin at a therapeutically effective rate;
(b) a backing on a skin distal surface of the reservoir; and
(c) means for maintaining the reservoir in fenoldopam transmitting and skin permeation enhancer transmitting relation with the skin;
characterized in that the composition comprises:
(i) 1 to 50 weight % of fenoldopam;
(ii) 1 to 50 weight % of the skin permeation enhancer; and
(iii)30 to 90 weight % of a polymeric carrier.

Preferably, the transdermal flux of fenoldopam is at least 1 *µ*g per hour per cm² of skin and the administration period is at least 6 hours.

The device may comprise a composition as defined above.

### BRIEF DESCRIPTION OF THE FIGURES

Figure 1 is a cross-section through a schematic perspective view of one embodiment of a transdermal therapeutic system according to the invention.
Figure 2 is a cross-section view through another embodiment of this invention prior to application to the skin.
Figure 3 is a cross-section view through another embodiment of this invention prior to application to the skin.
Figure 4 is a cross-section view through another embodiment of this invention prior to application to the skin.
Figure 5 depicts the release rate of fenoldopam base from polymer matrix formulations containing various permeation enhancers.
Figure 6 depicts the release rate of fenoldopam mesylate from polymer matrix formulations containing various permeation enhancers.
Figures 7 and 8 depict the flux of fenoldopam base and mesylate from polymer matrix formulations containing various permeation enhancers.
Figure 9 depicts the flux of fenoldopam base from polymer matrix formulations containing GML and dodecyl acetate (lauryl acetate).

### DETAILED DESCRIPTION OF THE INVENTION

According to this invention, it has been discovered that fenoldopam can be safely and efficaciously administered by a sustained release formulation. More specifically, it has been found that fenoldopam can be safely and efficaciously administered transdermally at a therapeutically effective rate to provide, among other things, treatment for hypertension, congestive heart failure, and acute renal failure when coadministered with a suitable permeation enhancer. The present invention provides novel compositions, devices, and methods for fenoldopam therapy with improved patient compliance to an individual in need of such therapy.

Therapeutic blood or plasma levels can be obtained from administration rates in the range of 20-1500 µg/hr, preferably about 60 - 1000 µg/hr. Representative *in vitro* skin fluxes of fenoldopam through human skin are in the range of about 5 ng/cm^{2 ·} hr - 5.5 µg/cm^{2·}hr, depending on the drug form, permeation enhancer, and adhesive.

This invention finds particular usefulness in administering fenoldopam across skin. According to the invention, fenoldopam is placed in fenoldopam transmitting relationship to an appropriate skin site, preferably in a pharmaceutically acceptable carrier thereof, and maintained in place for the desired administration period.

The fenoldopam and permeation enhancer are typically dispersed within a physiologically compatible matrix or carrier, as more fully described below, which may be applied directly to the skin as an ointment, gel, or cream. When used in the form of a liquid, ointment, lotion, cream or gel, it is preferable, although not required, to occlude the site of administration. Such compositions can also contain other permeation enhancers, stabilizers, dyes, diluents, pigments, vehicles, inert fillers, anti-irritants, excipients, gelling agents, vasoconstrictors, vasodilators, and other components of topical compositions as are known to the art.

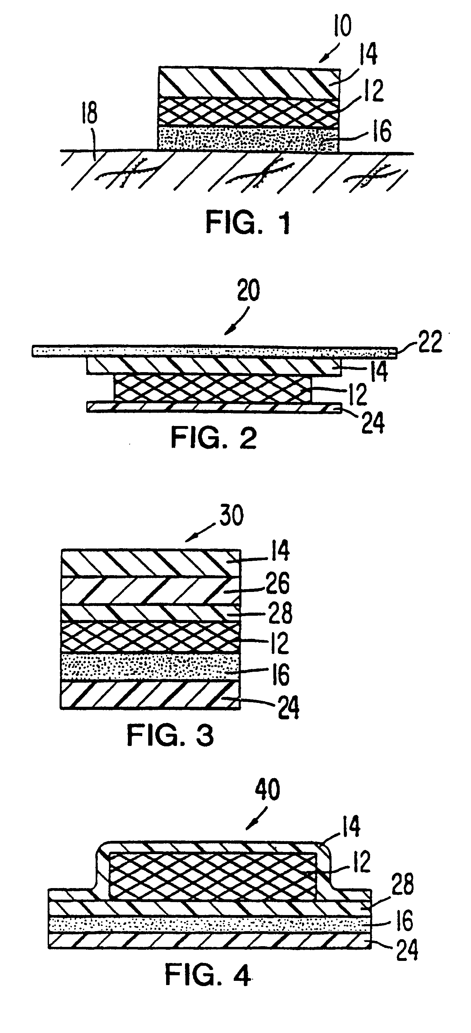

In other embodiments, fenoldopam would be administered from a transdermal delivery device as more fully described below. Examples of suitable transdermal delivery devices are illustrated in Figs. 1 - 4. In the figures, the same reference numbers are used throughout the different figures to designate the same or similar components. The figures are not drawn to scale.

Referring now to Figure 1, a preferred embodiment of a transdermal therapeutic system according to this invention comprises transdermal delivery device 10 comprising a reservoir 12, preferably in the form of a matrix containing fenoldopam, and a permeation enhancer dispersed therein. Reservoir 12 is sandwiched between a backing 14 and an in-line contact adhesive layer 16. The device 10 adheres to the surface of the skin 18 by means of the adhesive layer 16. The adhesive layer 16 may optionally contain the permeation enhancer and/or fenoldopam. A removable release liner (not shown in FIG. 1) is normally provided along the exposed surface of adhesive layer 16 and is removed prior to application of device 10 to the skin 18. Optionally, a rate-controlling membrane (not shown) may be present between the reservoir 12 and the adhesive layer 16. Additionally, a non-rate controlling tie layer membrane as disclosed in US Patent No. 5,635,203, incorporated herein in its entirety by reference, may be present between the reservoir 12 and adhesive 16 in any of the embodiments depicted in Figures 1-4.

Although the preferred embodiments of this invention utilize an in-line adhesive as is shown in Figure 1, other means for maintaining the system on the skin can be employed. Such means include a peripheral ring of adhesive outside the path of the drug from the system to the skin or the use of other fastening means such as buckles, belts, and elastic arm bands.

Alternatively, reservoir 12 may be in the form of a matrix containing fenoldopam and permeation enhancer dispersed within a suitable adhesive, preferably a pressure sensitive adhesive. Such pressure sensitive adhesives include, but are not limited to, polysiloxanes, polyacrylates, polyurethanes, acrylic adhesives including cross linked or non-crosslinked acrylic copolymers, vinyl acetate adhesives, ethylene vinylacetate copolymers, and natural or synthetic rubbers including polybutadienes, polyisoprenes, and polyisobutylene adhesives, and mixtures and graft copolymers thereof. The matrix formulations according to this embodiment comprise the adhesive containing fenoldopam and permeation enhancer, if present, laminated to a backing on one surface and to a release liner on the other. In addition to the fenoldopam and permeation enhancer, the matrix or carrier may also contain dyes, pigments, inert fillers, anti-irritants, excipients and other conventional components of pharmaceutical products or transdermal devices known to the art. For example, the matrix may also be provided with hydrophilic water absorbing and water soluble polymers known in the art such as polyvinyl alcohol and polyvinyl pyrrolidone individually or in combination. Other suitable water soluble and water absorbing polymers are known in the art, such as those disclosed in U.S. Patent No. 5,176,916, hereby incorporated in its entirety by reference.

Alternatively, as shown in FIG. 2, transdermal therapeutic device 20 may be attached to the skin of a patient by means of an adhesive overlay 22. Device 20 is comprised of reservoir 12 preferably in the form of a matrix containing fenoldopam and a permeation enhancer dispersed therein. A backing layer 14 is provided adjacent to one surface of reservoir 12. Adhesive overlay 22 maintains the device on the skin and may be fabricated together with, or provided separately from, the remaining elements of the device. With certain formulations, the adhesive overlay 22 may be preferable to the in-line contact adhesive 16 as shown in FIG. 1. Backing layer 14 is preferably slightly larger than reservoir 12, and in this manner prevents the materials in reservoir 12 from adversely interacting with the adhesive in overlay 22. Optionally, a rate-controlling membrane (not shown in FIG. 2) may be provided on the skin-proximal side of reservoir 12. A removable release liner 24 is also provided with device 20 and is removed just prior to application of device 20 to the skin.

In FIG. 3, transdermal delivery device 30 comprises a fenoldopam and permeation enhancer reservoir ("fenoldopam reservoir') 12 substantially as described with respect to FIG. 1. Permeation enhancer reservoir ("enhancer reservoir") 26 comprises the permeation enhancer dispersed throughout and contains fenoldopam at or below saturation, when in equilibrium with the fenoldopam reservoir 12. Enhancer reservoir 26 is preferably made from substantially the same matrix as is used to form fenoldopam reservoir 12. A rate-controlling membrane 28 for controlling the release rate of the permeation enhancer from enhancer reservoir 26 to fenoldopam reservoir 12 is placed between the two reservoirs. A rate-controlling membrane (not shown in FIG. 3) for controlling the release rate of the enhancer and/or fenoldopam from fenoldopam reservoir 12 to the skin may also optionally be utilized and would be present between adhesive layer 16 and reservoir 12.

The rate-controlling membrane may be fabricated from permeable, semipermeable or microporous materials which are known in the art to control the rate of agents into and out of delivery devices and having a permeability to the permeation enhancer lower than that of drug reservoir 12. Suitable materials include, but are not limited to, polyethylene, polyvinyl acetate, ethylene n-butyl acetate and ethylene vinyl acetate copolymers.

Superimposed over the permeation enhancer reservoir 26 of device 30 is a backing 14. On the skin-proximal side of reservoir 12 are an adhesive layer 16 and a removable liner 24 which would be removed prior to application of the device 30 to the skin.

In the embodiments of FIGS. 1, 2 and 3, the carrier or matrix material of the reservoirs has sufficient viscosity to maintain its shape without oozing or flowing. If, however, the matrix or carrier is a low-viscosity flowable material such as a liquid or a gel, the composition can be fully enclosed in a pouch or pocket, as known to the art from US Pat. No. 4,379,454 (noted above), for example, and as illustrated in FIG. 4. Device 40 shown in FIG. 4 comprises a backing member 14 which serves as a protective cover for the device, imparts structural support, and substantially keeps components in device 40 from escaping the device. Device 40 also includes reservoir 12, which contains the fenoldopam and permeation enhancer and bears on its surface distant from backing member 14, a rate-controlling membrane 28 for controlling the release of fenoldopam and/or permeation enhancer from device 40. The outer edges of backing member 14 overlay the edges of reservoir 12 and are joined along the perimeter with the outer edges of the rate-controlling membrane 28 in a fluid-tight arrangement. This sealed reservoir may be effected by pressure, fusion, adhesion, an adhesive applied to the edges, or other methods known in the art. In this manner, reservoir 12 is contained wholly between backing member 14 and rate-controlling membrane 28. On the skin-proximal side of rate-controlling membrane 28 are an adhesive layer 16 and a removable liner 24 which would be removed prior to application of the device 40 to the skin.

In an alternative embodiment of device 40 of FIG. 4, reservoir 12 contains the permeation enhancer and contains fenoldopam at or below saturation. The fenoldopam and an additional amount of permeation enhancer are present in adhesive layer 16, which acts as a separate reservoir.

Fenoldopam can be administered to human skin by direct application to the skin in the form of an ointment, gel, cream or lotion, for example, but are preferably administered from a skin patch or other known transdermal delivery device which contains a saturated or unsaturated formulation of the fenoldopam and enhancer. The formulation may be aqueous or non-aqueous. The formulation should be designed to deliver the fenoldopam and any anti-irritant and/or enhancer at the necessary fluxes. Aqueous formulations typically comprise water or water/ethanol and about 1-5 wt% of a gelling agent, an example being a hydrophilic polymer such as hydroxyethylcellulose or hydroxypropylcellulose. When using aqueous formulations, it is preferable to maintain the pH at less than about 5.5, more preferably between about pH 2 - 4.5 in order to provide a stable fenoldopam formulation. Typical non-aqueous gels are comprised of silicone fluid or mineral oil. Mineral oil-based gels also typically contain 1-2 wt% of a gelling agent such as colloidal silicon dioxide. The suitability of a particular gel depends upon the compatibility of its constituents with the fenoldopam, anti-irritant, and the permeation enhancer in addition to any other components in the formulation.

The reservoir matrix should be compatible with fenoldopam, the permeation enhancer, and any carrier therefor. The term "matrix" as used herein refers to a well-mixed composite of ingredients. When using an aqueous formulation, the reservoir matrix is preferably a hydrophilic polymer, e.g., a hydrogel.

When using a non-aqueous formulation, the reservoir matrix is preferably composed of a hydrophobic polymer. Suitable polymeric matrices are well known in the transdermal drug delivery art, and examples are listed in the above-named patents previously incorporated herein by reference. A typical laminated. system would consist essentially of a polymeric membrane and/or matrix such as ethylene vinyl acetate (EVA) copolymers, such as those described in US Pat. No. 4,144,317, preferably having a vinyl acetate (VA) content in the range of from about 9% up to about 60% and more preferably about 9% to 40% VA. Polyisobutylene/oil polymers containing from 4-25% high molecular weight polyisobutylene and 20-81% low molecular weight polyisobutylene with the balance being an oil such as mineral oil or polybutene may also be used as the matrix material.

The amount of fenoldopam present in the therapeutic device and required to achieve an effective therapeutic result depends on many factors, such as the minimum necessary dosage of the fenoldopam for the particular indication being treated; the solubility and permeability of the matrix, taking into account the presence of permeation enhancer, of the adhesive layer and of the rate-controlling membrane, if present; and the period of time for which the device will be fixed to the skin. The minimum amount of fenoldopam is determined by the requirement that sufficient quantities of fenoldopam must be present in the device to maintain the desired rate of release over the given period of application. The maximum amount for safety purposes is determined by the requirement that the quantity of fenoldopam present must not support a rate of release that reaches toxic levels.

The fenoldopam may be present in the matrix or carrier at a concentration at or below saturation. An excess amount of fenoldopam above saturation may be included in the matrix or carrier, the amount of excess being a function of the desired length of the delivery period of the system. Fenoldopam may be present at a level below saturation without departing from this invention as long as it is continuously administered to the skin site at a therapeutic rate and for a period of time sufficient to deliver a therapeutically effective amount of fenoldopam that provides the desired therapeutic result.

The permeation enhancer useful in the present invention is selected from those compounds which are compatible with fenoldopam and which provide enhanced skin permeation to the drug when it is administered together with the drug to the skin of a user. Additionally, the permeation enhancer must not adversely interact with the adhesive of the in-line contact adhesive layer if one is present Examples of permeation enhancers are disclosed in the patents cited above previously incorporated by reference and can be selected from, but are not limited to, fatty acids, monoglycerides of fatty acids such as glycerol monolaurate, glycerol monooleate, glycerol monocaprate, glycerol monocaprylate, or glycerol monolinoleate; lactate esters of fatty acids such as lauryl lactate, cetyl lactate, and myristyl lactate; acyl lactylates such as caproyl lactylic acid; esters of fatty acids having from about 10 to about 20 carbon atoms, including, but not limited to, isopropyl myristate, and ethyl palmitate; alkyl laurates such as methyl laurate; dimethyl lauramide; lauryl acetate; monoalkyl ethers of polyethyleneglycol and their alkyl or aryl carboxylic acid esters and carboxymethyl ethers such as polyethylene glycol-4 lauryl ether (Laureth-4) and polyethylene glycol-2 lauryl ether (Laureth-2); polyethylene glycol monolaurate; myristyl sarcosine; Myreth-3; and lower C₁₋₄ alcohols such as isopropanol and ethanol, alone or in combinations of one or more.

A preferred permeation enhancer according to this invention comprises a monoglyceride of a fatty acid together with a suitable cosolvent, including, but not limited to, lauryl acetate as disclosed in WO 96/40259 and esters of C₁₀ - C₂₀ fatty acids such as lauryl lactate, ethyl palmitate, and methyl laurate. Ethyl palmitate has been found to be particularly desirable as it is obtainable at a high degree of purity, thus providing a purer and better defined permeation enhancer and a system which is more readily characterized. According to a particularly preferred embodiment, the permeation enhancer comprises glycerol monolaurate (GML) and ethyl palmitate within the range of 1-25 wt% and 1-20 wt%, respectively, at a ratio of GML/ ethyl palmitate within the range of 0.5 - 5.0, preferably 1.0 - 3.5. A particularly preferred embodiment comprises 20 wt% GML and 12 wt% ethyl palmitate.

Another embodiment is directed to the use of myristyl sarcosine as a permeation enhancer for fenoldopam mesylate. In general, formulations comprising fenoldopam base were found to be more permeable through the skin as compared to formulations comprising pharmaceutically acceptable salts of fenoldopam such as fenoldopam mesylate. However, formulations comprising myristyl sarcosine as a permeation enhancer for fenoldopam mesylate were found to exhibit higher transdermal fluxes than formulations of the base. Additionally, fenoldopam mesylate when administered transdermally did not exhibit the long lag period observed with fenoldopam base. Thus, according to this embodiment, transdermal compositions, devices, and methods are provided comprising a pharmaceutically acceptable salt of fenoldopam, preferably fenoldopam mesylate; together with a permeation enhancer for the fenoldopam salt, preferably myristyl sarcosine, in order to transdermally administer fenoldopam at therapeutically effective rates and lowered lag time.

The permeation-enhancing mixture is dispersed through the matrix or carrier, preferably at a concentration sufficient to provide permeation-enhancing amounts of enhancer in the reservoir throughout the anticipated administration period. Where there is an additional, separate permeation enhancer matrix layer as well, as in FIGS. 3 and 4, the permeation enhancer normally is present in the separate reservoir in excess of saturation.

According to another preferred embodiment, an anti-irritant is dispersed throughout the matrix or carrier, preferably at a concentration sufficient to deliver anti-irritant to the skin in an amount effective to reduce skin irritation throughout the anticipated administration period. The anti-irritant is preferably present in excess of saturation in order to ensure that the anti-irritant is continuously administered with the fenoldopam and continues to be present as long as any fenoldopam is present in the epidermis. Suitable anti-irritants include, but are not limited to, methyl nicotinate as disclosed in US Patent No. 5,451,407, corticosteroids, and buffering agents including ascorbic acid and acetic acid. Such anti-irritants are known in the art as seen in the above cited patents previously incorporated by reference.

For example, if a corticosteroid is used as the anti-irritant, it is preferably administered at a flux within the range of 0.1 - 5.0 µg/cm^{2·}hr. Hydrocortisone is a preferred corticosteroid and is present in an amount of about 1 - 5 wt%. The total amount of hydrocortisone administered is not to exceed 5 mg / 24 hour in order to avoid possible systemic effects. Hydrocortisone esters such as hydrocortisone acetate are also suitable. More potent corticosteroids may not require a permeation enhancer as hydrocortisone and hydrocortisone acetate do. However, the advantages of hydrocortisone or its esters such as hydrocortisone acetate is that they are approved for over-the-counter use. This invention contemplates the use of any corticosteroid in addition to hydrocortisone and includes, without limitation, beclomethasone, betamethasone, benzoid, betamethasone dipropionate, betamethasone valerate, clobetasol propionate, clobetasol butyrate, desonide, dexamethasone, fluocinonide, prednisolone, and triamcinolone, for example.

Because of the wide variation in skin permeability from individual to individual and from site to site on the same body, it may be preferable that the fenoldopam, anti-irritant, and/or permeation enhancer, be administered from a rate-controlled transdermal delivery device. Rate control can be obtained either through a rate-controlling membrane as described in U.S. Patent No. 3,797,494 listed above, or through an adhesive or both as well as through other means known in the art.

A certain amount of fenoldopam may bind reversibly to the skin, and it is accordingly preferred that the skin-contacting layer of the device include this amount of fenoldopam as a loading dose.

The surface area of the device of this invention can vary from about 1-200 cm². A typical device, however, will have a surface area within the range of about 5-60 cm², preferably about 20 cm².

The devices of this invention can be designed to deliver fenoldopam effectively for an extended time period of from several hours up to 7 days or longer. Seven days is generally the maximum time limit for application of a single device because the adverse effects of occlusion of a skin site increases with time and the normal cycle of sloughing and replacement of the skin cells occurs in about 7 days.

Preferably, a device for the transdermal administration of fenoldopam, at a therapeutically effective rate, comprises:
(a) a reservoir comprising:
   (i) 1-50% by weight fenoldopam,
   (ii) 1-50% by weight of a permeation enhancer,
   (iii) 30 to 90% by weight of a polymeric carrier;
(b) a backing behind the skin-distal surface of the reservoir; and
(c) means for maintaining the reservoir in fenoldopam - transmitting relation with the skin.

More preferably, a device for the transdermal administration of fenoldopam, at a therapeutically effective rate, comprises:
(a) a reservoir comprising:
   (i) 1 - 50% by weight fenoldopam,
   (ii) 5 - 40% by weight of a permeation enhancer,
   (iii) 30 - 90% by weight of a polymeric carrier;
(b) a backing behind the skin-distal surface of the reservoir; and
(c) means for maintaining the reservoir in fenoldopam- transmitting relation with the skin.

Most preferably, a device for the transdermal administration of fenoldopam, at a therapeutically effective rate, comprises:
(a) a reservoir comprising:
   (i) 5 - 50% by weight fenoldopam,
   (ii) 5 - 40% by weight of a permeation enhancer comprising a monoglyceride and a fatty acid ester,
   (iii) 30 - 90% by weight of a polymeric carrier;
(b) a backing behind the skin-distal surface of the reservoir; and
(c) means for maintaining the reservoir in fenoldopam - transmitting relation with the skin.

The backing may be flexible or nonflexible and may be a breathable or occlusive material. Suitable materials include, without limitation, polyethylene, polyurethane, polyester, ethylene vinyl acetate, acrylonitrile, cellophane, cellulose acetate, cellulosics, ethylcellulose, ethylene vinyl alcohol, plasticized vinylacetate-vinylchloride copolymers, polyethylene terephthalate, nylons, rayon, polypropylene, polyvinyl alcohol, polyvinyl chloride, metalized polyester films, polyvinylidene chloride, polycarbonate, polystyrene, and aluminum foil. The backing may be a multi-laminate film.

The means for maintaining the reservoir in drug and permeation enhancer transmitting relation with the skin is preferably a pressure sensitive adhesive including, but not limited to, polyisobutylene adhesives, silicone adhesives, and acrylate adhesives known in the art including copolymers and graft copolymers thereof. A further embodiment of the invention is directed to including in the adhesive a small percentage, e.g., from about 1 to about 5 wt% of fenoldopam to assure an appropriate initial release rate.

The aforementioned patents describe a wide variety of materials which can be used for fabricating various layers or components of the transdermal fenoldopam delivery systems according to this invention. This invention, therefore, contemplates the use of materials other than those specifically disclosed herein including those which may become hereafter known to the artist capable of performing the necessary functions.

The invention is also directed to a method of continuously administering fenoldopam to a patient at a therapeutically effective rate over an administration period in order to administer a therapeutically effective amount and achieve and maintain therapeutic blood or plasma levels in a patient.

A preferred embodiment of the present invention comprises a method of treating acute or chronic renal failure. According to this embodiment, about 1 - 6 mg of fenoldopam, preferably 1.5 - 4 mg, most preferably 2 - 3 mg, are delivered daily by the compositions, devices, and methods disclosed above. To achieve this result, fenoldopam is delivered at a therapeutic rate within a range of about 20 - 5500 µg/hr, preferably about 40 - 1500 µg/hr, most preferably 60 - 600 µg/hr from a reasonably sized transdermal delivery device having a surface area of less than about 60 cm² for the treatment period, usually about 6 hours to 5 days, preferably 24 - 72 hours.

The length of time of fenoldopam presence and the total amount of fenoldopam in the plasma can be changed following the teachings of this invention to provide different treatment regimens. Thus, they can be controlled by the amount of time during which exogenous fenoldopam is delivered transdermally to an individual or animal and the rate at which it is administered.

Having thus generally described our invention, the following specific examples describe preferred embodiments thereof but are not intended to limit the invention in any manner.

### EXAMPLE 1

About 3 grams of fenoldopam mesylate was weighed in a 250 ml beaker and dissolved in 120 ml warm distilled water (or to a saturated solution). 3M Na₂CO₃ solution was added to the fenoldopam solution drop by drop until the solution reached pH 8.5. The whitened Na₂CO₃ solution (converted fenoldopam base) was transferred to a Buchner funnel lined with Whatman #1 filter paper, washed several times with distilled water and vacuum dried at about 65 C. The dried fenoldopam base was transferred into a tared vial and weighed.

Transdermal flux of fenoldopam base and mesylate was measured from polymer matrices containing drug alone and from matrices containing chemical permeation enhancers singly or in combination. Drug reservoirs were prepared by mixing fenoldopam base or mesylate, ethylene vinylacetate (EVA)(USI Chemicals, Illinois) having a vinyl acetate content of 40%, and dodecyl acetate (DA) (Inoue Perfumery Mfg. Co. LTD, Tokyo, Japan), glycerol monolaurate (GML) (Danisco Ingredients), polyvinyl pyrrolidone (PVP) (XL-10, ISP Technologies, Inc., Calvert City, KY), lauramide diethanolamine (LDEA), Laureth-2, Laureth-4, Myreth-3, myristyl sarcosine, glycerol monocaprate (GMC), and/or caproyl lactylic acid (CLA) (American Ingredient Co., Grand Viejo, CA) in the amounts set forth in Table 1. The resulting mix was then calendered to a 5 mil thickness between 2 release liners. The drug reservoir was then heat laminated to a Medpar® backing on one surface and a 3M acrylate adhesive on the other. Circular systems were cut with a stainless steel punch.

**Table 1**

| Fenoldopam Base and Mesylate Formulations | | |
|---|---|---|
| **Formulation No.** | **Composition** | **Weight Percentages** |
| 1 (control) | fenoldopam base/EVA | 15/85 |
| 2 | fenoldopam base/EVA/GMUML/PVP | 15/37/20/12/16 |
| 3 | fenoldopam base/EVA/Laureth-4 | 15/65/20 |
| 4 | fenoldopam base/EVA/DML | 15/65/20 |
| 5 | fenoldopam base/EVA/MS | 15/65/20 |
| 6 | fenoldopam base/EVA/CLA/LDEA | 15/50/20/15 |
| 7 | fenoldopam base/EVA/Myreth-3 | 15/65/20 |
| 8 | fenoldopam base/EVA/Laureth-2 | 15/65/20 |
| 9 | fenoldopam base/EVA/GMC | 15/65/20 |
| 10 | fenoldopam mesylate/EVA | 15/85 |
| 11 | fenoldopam mesylate /EVA/GML/ML/PVP | 15/37/20/12/16 |
| 12 | fenoldopam mesylate /EVA/Laureth-4 | 15/65/20 |
| 13 | fenoldopam mesylate /EVA/DML | 15/65/20 |
| 14 | fenoldopam mesylate /EVA/MS | 15/65/20 |
| 15 | fenoldopam mesylate /EVA/CLA/LDEA | 15/50/20/15 |
| 16 | fenoldopam mesylate /EVA/Myreth-3 | 15/65/20 |
| 17 | fenoldopam mesylate /EVA/Laureth-2 | 15/65/20 |
| 18 | fenoldopam mesylate /EVA/GMC | 15/65/20 |

Release of fenoldopam from transdermal systems into aqueous medium was measured over 24 hours at 35° C. The release liner of the matrix system was then removed and each system had its exposed edges masked. The systems were then mounted on a Teflon® holder of a release rate rod using nylon mesh and nickel wire. A known volume of receptor solution (30 ml 0.01 M phosphate buffer solution at pH 2.5) was then placed in a test tube and equilibrated at 35°C. The test tube was placed in a water bath and maintained at 35°C. The Teflon rod with attached system was then reciprocated within the test tube by attaching the rod to a motor which caused constant vertical mixing.

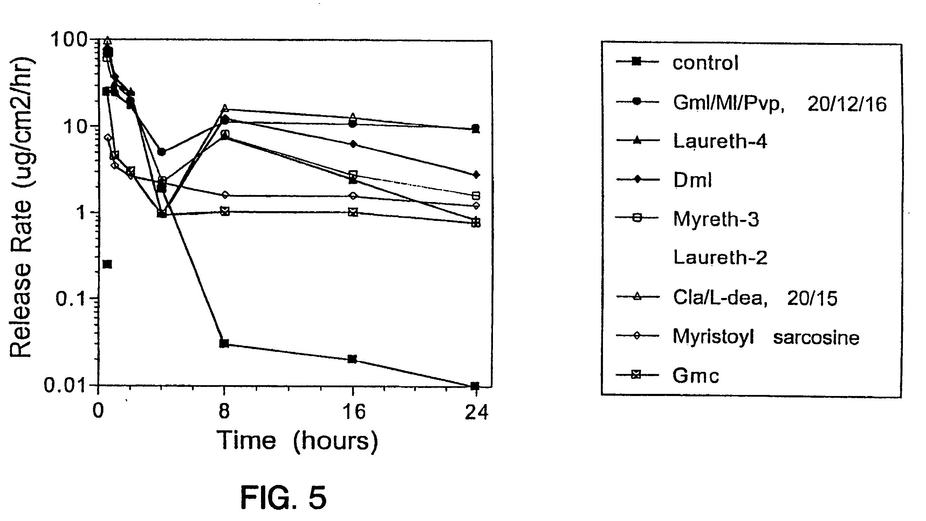

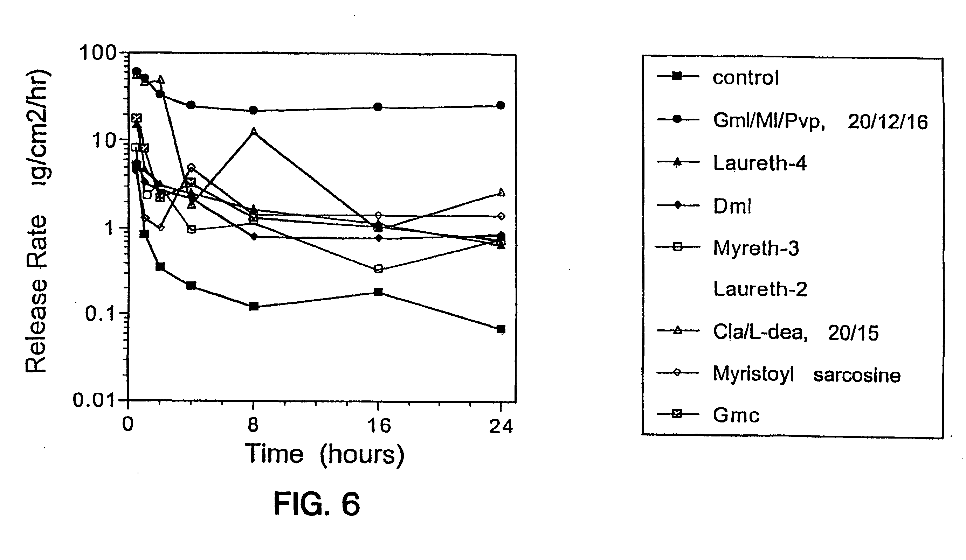

At given time intervals, the entire receptor solution was removed from the test tubes and replaced with an equal volume of fresh receptor solutions previously equilibrated at 35°C. The receptor solutions were stored in capped vials at 4 °C until assayed for fenoldopam content by HPLC. From the drug concentration and the volume of the receptor solutions, the area of the system and the time interval, the release rate of the drug from the system was calculated as follows: (drug concentration X volume of receptor)/(area x time) = release rate (µg/cm^{2 ·} hr). Release rates for the fenoldopam base and mesylate formulations are depicted in Figures 5 and 6, respectively.

### EXAMPLE 2

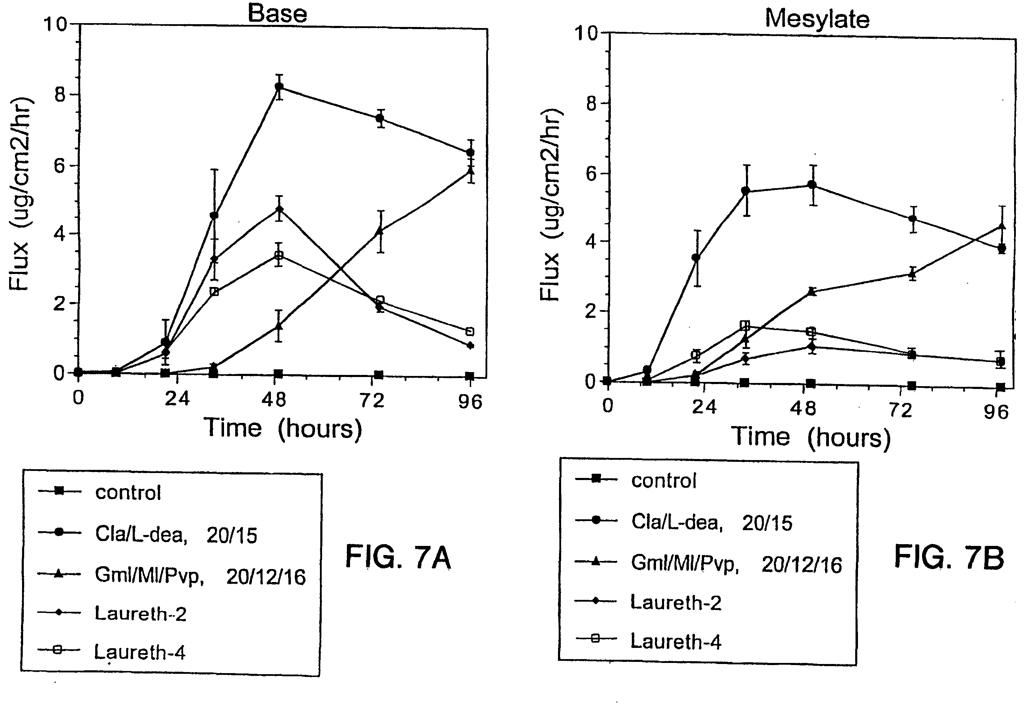

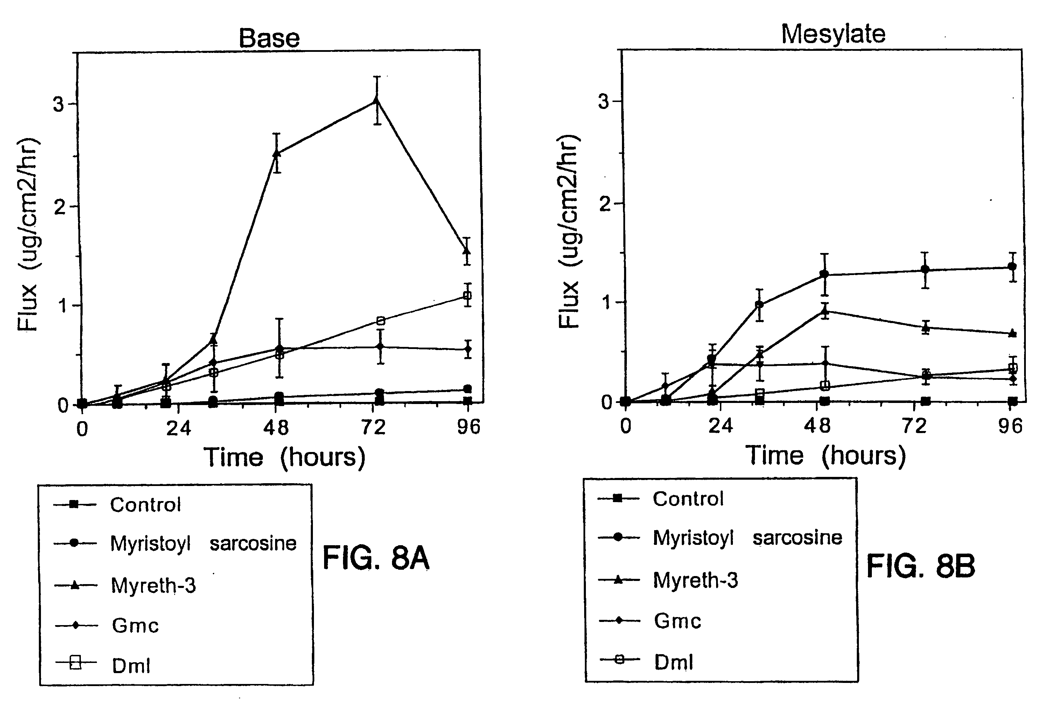

Transdermal systems set forth in Table 1 were prepared according to Example 1 and used to measure permeation through human cadaver skin. Circular pieces of heat stripped human epidermis were blotted dry just prior to use and the stratum corneum surface of the epidermis was applied to the fenoldopam releasing side of the system. The edges of epidermis were then folded around the system so that none of the system edge was exposed to the receptor solution. Fenoldopam permeation through the epidermis was then measured according to the procedure set forth in Example 1. The receptor compartment was filled with a known volume of 0.01 M KH₂PO₄/K₂HPO₄ which was adjusted to pH 2.5 with 10% H₃PO₄ buffer solution previously equilibrated at 35 °C. Figures 7 and 8 depict the *in vitro* transdermal flux of fenoldopam through human epidermis with various permeation enhancers.

### EXAMPLE 3

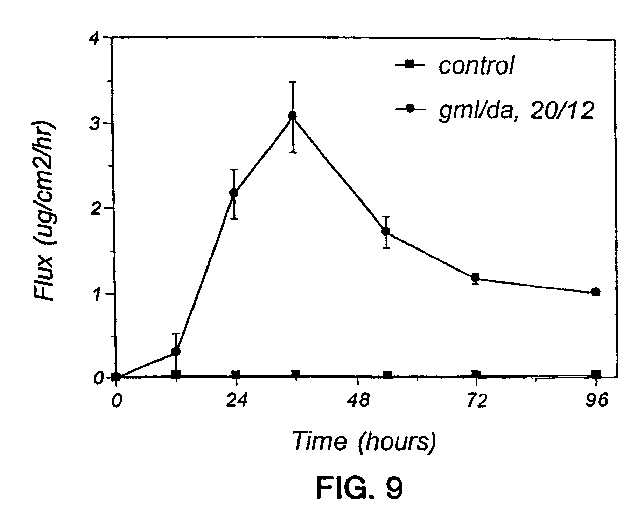

Transdermal systems set forth in Table 2 were prepared according to the procedure set forth in Example 1 and used to measure permeation through human cadaver skin. Circular pieces of heat stripped human epidermis were blotted dry just prior to use and the stratum comeum surface of the epidermis was applied to the fenoldopam releasing side of the system. The edges of epidermis were then folded around the system so that none of the system edge was exposed to the receptor solution. Fenoldopam permeation through the epidermis was then measured according to the procedure set forth in Example 1. The receptor compartment was filled with a known volume of 0.01 M KH₂PO₄/K₂HPO₄ which was adjusted to pH 2.5 with 10% H₃PO₄ buffer solution previously equilibrated at 35 °C. Figure 9 depicts the mean in vitro transdermal flux of fenoldopam through human epidermis with various permeation enhancers.

**Table 2**

| Fenoldopam Base Formulations | | |
|---|---|---|
| **Formulation No.** | **Composition** | **Weight Percentages** |
| 1 (control) | fenoldopam base/EVA | 15/85 |
| 2 | fenoldopam base/EVA/GML/DA | 15/53/20/12 |

Having thus generally described our invention and described certain specific embodiments thereof, including the embodiments that applicants consider the best mode of practicing their invention, it should be readily apparent that various modifications to the invention may be made by workers skilled in the art without departing from the scope of this invention which is limited only by the following claims.

## Claims

1. A composition of matter for administration of fenoldopam through intact skin, the composition comprising fenoldopam and a skin permeation enhancer in a carrier, the skin permeation enhancer being present in an amount sufficient to enhance transdermal flux of fenoldopam compared to transdermal flux of fenoldopam from a similar composition having no skin permeation enhancer, the composition being effective for sustained delivery of fenoldopam through the skin at a therapeutically effective rate, **characterized in that** the composition comprises:
(a) 1 to 50 weight % of fenoldopam;
(b) 1 to 50 weight % of the skin permeation enhancer; and
(c) 30 to 90 weight % of a polymeric carrier.

2. A composition according to claim 1, wherein the transdermal flux of fenoldopam is at least 1 *µ*g per hour per cm² of skin and the administration period is at least 6 hours.

3. A composition according to either claim 1 or 2 wherein the fenoldopam comprises a pharmaceutically acceptable salt.

4. A composition according to claim 3 wherein the salt is fenoldopam mesylate.

5. A composition according to any preceding claim wherein the permeation enhancer comprises a monoglyceride, myristyl sarcosine, or a mixture thereof.

6. A composition according to claim 5 further comprising a cosolvent selected from fatty acid esters, caproyl lactylic acid, lauroyl lactylic acid, and dimethyl lauramide.

7. A composition according to claim 6 wherein the monoglyceride is glycerol monolaurate and the cosolvent is selected from dodecyl acetate, lauryl lactate, isopropyl myristate, ethyl palmitate, and methyl laurate.

8. A composition according to any preceding claim comprising 5 to 40 weight % fenoldopam base and 5 to 50 weight % of a skin permeation enhancer comprising a monoglyceride and a fatty acid ester.

9. A device (10;20;30;40) for the transdermal administration of fenoldopam at a therapeutically effective rate, comprising:
(a) a reservoir (12) comprising a composition comprising fenoldopam and a skin permeation enhancer in a carrier, the skin permeation enhancer being present in an amount sufficient to enhance transdermal flux of fenoldopam compared to transdermal flux of fenoldopam from a similar composition having no skin permeation enhancer, the composition being effective for sustained delivery of fenoldopam through the skin at a therapeutically effective rate;
(b) a backing (14) on a skin distal surface of the reservoir; and
(c) means (16;22) for maintaining the reservoir in fenoldopam transmitting and skin permeation enhancer transmitting relation with the skin;
**characterized in that** the composition comprises:
(i) 1 to 50 weight % of fenoldopam;
(ii) 1 to 50 weight % of the skin permeation enhancer; and
(iii) 30 to 90 weight % of a polymeric carrier.

10. A device according to claim 9 comprising the composition claimed in any of claims 3 to 8.

11. A device according to claim 9 or 10 wherein the transdermal flux of fenoldopam is at least 1 µg per hour per cm² of skin and the administration period is at least 6 hours.

## Patentansprüche

1. Substanzzusammensetzung zur Verabreichung von Fenoldopam durch intakte Haut, wobei die Zusammensetzung Fenoldopam und einen Hautdurchlässigkeitsverbesserer in einem Träger umfasst, und der Hautdurchlässlgkeltsverbesserer in einer Menge vorhanden ist, die ausreicht, um den transdermalen Fenoldopamfluß im Vergleich zum transdermalen Fenoldopamfluß aus einer ähnlichen Zusammensetzung ohne Hautdurchlässigkeitsverbesserer zu verstärken, wobei die Zusammensetzung für die verzögerte Abgabe von Fenoldopam durch die Haut mit einer therapeutisch effektiven Rate wirksam ist, **dadurch gekennzeichnet, daß** die Zusammensetzung umfasst:
a) 1 bis 50 Gew.-% Fenoldopam;
b) 1 bis 50 Gew.-% Hautdurchlässigkeitsverbesserer;
und
c) 30 bis 90 Gew.-% eines Polymerträgers.

2. Zusammensetzung nach Anspruch 1, wobei der transdermale Fenoldopamfluß mindestens 1 µg pro Stunde pro cm² Haut, und die Verabreichungsdauer mindestens 6 Stunden beträgt.

3. Zusammensetzung nach Anspruch 1 oder 2, worin das Fenoldopam ein pharmazeutisch geeignetes Salz umfasst.

4. Zusammensetzung nach Anspruch 3, worin das Salz Fenoldopammesylat ist.

5. Zusammensetzung nach einem der vorhergehenden Ansprüche, wobei der Durchlässigkeltverbesserer ein Monogylcerid, Myristylsarkosin und ein Gemisch davon umfasst.

6. Zusammensetzung nach Anspruch 5, die weiterhin ein Hilfslösungsmittel umfasst, das ausgewählt ist aus Fettsäureestern, Capryllactylsäure, Lauroyllactylsäure und Dimethyllauramid.

7. Zusammensetzung nach Anspruch 6, worin das Monoglycerid Glycerinmonolaurat Ist, und das Hitfslösungsmittel ausgewählt ist aus Dodecylacetat, Lauryllactat, Isopropylmyristat, Ethylpalmitat und Methyllaurat.

8. Zusammensetzung nach einem der vorhergehenden Ansprüche, die 5 bis 40 Gew.-% Fenoldopambase und 5 bis 50 Gew.-% eines Hautdurchlässigkeitsverbesserers, ein Monoglycerid und einen Fettsäureester umfasst.

9. Vorrichtung (10; 20; 30; 40) für die transdermale Verabreichung von Fenoldopam mit einer therapeutisch effektiven Rate, welche umfasst:
(a) Reservoir (12), umfassend eine Zusammensetzung, die Fenoldopam und einen Hautdurchlässigkeitsverbesserer in einem Träger umfasst, wobei der Hautdurchlässigkeitsverbesserer in einer Menge vorhanden ist, die ausreicht, um den transdermalen Fenoldopamfluß im Vergleich zum transdermalen Fenoldopamfluß aus einer ähnlichen Zusammensetzung ohne Hautdurchlässigkeitsverbesserer zu verstärken, wobei die Zusammensetzung für die verzögerte Abgabe von Fenoldopam durch die Haut mit einer therapeutisch effektiven Rate wirksam ist;
(b) eine Stützschicht (14) auf einer von der Haut abgewandten Seite des Reservoirs; und
(c) Vorrichtungen (16; 22), um das Reservoir in einem Übertragungsverhältnis von Fenoldopam und Hautdurchlässigkeitsverbesserer zu halten;
**dadurch gekennzeichnet, daß** die Zusammensetzung umfasst:
(i) 1 bis 50 Gew.-% Fenoldopam
(ii) 1 bis 50 Gew.-% des Hautdurchlässigkeitsverbesserers;
und 30 bis 90 Gew.-% Polymerträger.

10. Vorrichtung nach Anspruch 9, umfassend die Zusammensetzung nach einem der Ansprüche 3 bis 8.

11. Vorrichtung nach Anspruch 9 oder 10, worin der transdermale Fenoldopamfluß mindestens 1 µg Stunde pro cm² der Haut, und die Verabreichungsdauer mindestens 6 Stunden beträgt.

## Revendications

1. Composition de matière pour l'administration de fénoldopam à travers la peau intacte, la composition comprenant du fénoldopam et un agent facilitant la pénétration à travers la peau, dans un support, l'agent facilitant la pénétration à travers la peau étant présent dans une quantité suffisante pour augmenter le flux transdermique de fénoldopam par comparaison avec le flux transdermique de fénoldopam provenant d'une composition analogue n'ayant pas d'agent facilitant la pénétration à travers la peau, la composition étant efficace pour l'administration entretenue de fénoldopam à travers la peau à une vitesse thérapeutiquement efficace, **caractérisée par le fait que** la composition comprend :
(a) 1 à 50 % en poids de fénoldopam ;
(b) 1 à 50 % en poids de l'agent facilitant la pénétration à travers la peau ; et
(c) 30 à 90 % en poids d'un support polymère.

2. Composition selon la revendication 1, dans laquelle le flux transdermique de fénoldopam est d'au moins 1 µg par heure par cm² de peau, et la période d'administration est d'au moins 6 heures.

3. Composition selon l'une des revendications 1 ou 2, dans laquelle le fénoldopam comprend un sel pharmaceutiquement acceptable.

4. Composition selon la revendication 3, dans laquelle le sel est le mésylate de fénoldopam.

5. Composition selon l'une quelconque des revendications précédentes, dans laquelle l'agent facilitant la pénétration comprend un monoglycéride, la myristyl sarcosine, ou un mélange de ceux-ci.

6. Composition selon la revendication 5, comprenant en outre un cosolvant choisi parmi les esters d'acides gras, l'acide caproyl lactylique, l'acide lauroyl lactylique, et le diméthyl lauramide.

7. Composition selon la revendication 6, dans laquelle le monoglycéride est le monolaurate de glycérol et le cosolvant est choisi parmi l'acétate de dodécyle, le lactate de lauryle, le myristate d'isopropyle, le palmitate d'éthyle et le laurate de méthyle.

8. Composition selon l'une quelconque des revendications précédentes, comprenant 5 à 40 % en poids de fénoldopam base et 5 à 50% en poids d'un agent facilitant la pénétration à travers la peau comprenant un monoglycéride est un ester d'acide gras.

9. Dispositif (10; 20; 30; 40) pour l'administration transdermique de fénoldopam à une vitesse thérapeutiquement efficace, comprenant :
(a) un réservoir (12) comprenant une composition comprenant du fénoldopam et un agent facilitant la pénétration à travers la peau, dans un support, l'agent facilitant la pénétration à travers la peau étant présent dans une quantité suffisante pour augmenter le flux transdermique de fénoldopam par comparaison avec le flux transdermique de fénoldopam provenant d'une composition analogue n'ayant pas d'agent facilitant la pénétration à travers la peau, la composition étant efficace pour l'administration entretenue de fénoldopam à travers la peau à une vitesse thérapeutiquement efficace ;
(b) un support (14) sur une surface du réservoir distale par rapport à la peau; et
(c) un moyen (16; 22) pour maintenir le réservoir en relation de transmission de fénoldopam et de transmission d'agent facilitant la pénétration à travers la peau, avec la peau;
**caractérisé par le fait que** la composition comprend :
(i) 1 à 50 % en poids de fénoldopam;
(ii) 1 à 50 % en poids de l'agent facilitant la pénétration à travers la peau; et
(iii) 30 à 90 % en poids d'un support polymère.

10. Dispositif selon la revendication 9, comprenant la composition telle que définie à l'une quelconque des revendications 3 à 8.

11. Dispositif selon l'une des revendications 9 et 10, dans lequel le flux transdermique de fénoldopam est d'au moins 1 µg par heure par cm² de peau et la période d'administration est d'au moins 6 heures.
